(19) **Europäisches Patentamt / European Patent Office / Office européen des brevets**

(11) **EP 4 714 423 A1**

(12) **EUROPEAN PATENT APPLICATION**
published in accordance with Art. 153(4) EPC

(43) Date of publication:
**25.03.2026 Bulletin 2026/13**

(21) Application number: **24807244.9**

(22) Date of filing: **15.05.2024**

(51) International Patent Classification (IPC):
**A61K 8/29** (2006.01)   **A61Q 17/04** (2006.01)
**C01G 23/053** (2006.01)

(52) Cooperative Patent Classification (CPC):
**A61K 8/29; A61Q 17/04; C01G 23/053**

(86) International application number:
**PCT/JP2024/018030**

(87) International publication number:
**WO 2024/237294 (21.11.2024 Gazette 2024/47)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **16.05.2023   JP 2023080841**

(71) Applicant: **Tayca Corporation
Osaka-shi,
Osaka 551-0022 (JP)**

(72) Inventors:
- **SHIBATA Kazuya
Osaka-shi, Osaka 551-0022 (JP)**
- **EJIRI Kazumasa
Osaka-shi, Osaka 551-0022 (JP)**

(74) Representative: **Kador & Partner Part mbB
Corneliusstraße 15
80469 München (DE)**

(54) **SURFACE-TREATED POWDER AND COSMETIC USING SAME**

(57)   Provided is surface-treated powder of a fine particulate titanium oxide, which is capable of enhancing effect to suppress photoactivity of the fine particulate titanium oxide and enhancing stability of a pharmaceutical preparation. The surface-treated powder includes the fine particulate titanium oxide as powder to be surface-treated; and a fatty acid, and a value of a product of an average primary particle diameter (nm) of the fine particulate titanium oxide and a ratio of a mass of the fatty acid (in terms of the fatty acid) to a mass of the fine particulate titanium oxide is 1.5 or more and 3.7 or less.

EP 4 714 423 A1

**Description**

TECHNICAL FIELD

The present invention relates generally to surface-treated powder of a fine particulate titanium oxide and, in particular, to surface-treated powder of a fine particulate titanium oxide contained in a cosmetic and a cosmetic using the powder.

BACKGROUND ART

A fine particulate titanium oxide has high UV-B protective effect and is blended in many cosmetics. Since the fine particulate titanium oxide has high photoactivity, in general, the fine particulate titanium oxide is treated by hydrous silica or an aluminum hydroxide for the purpose of suppressing the photoactivity. In addition, in order to increase dispersibility into an oil agent, the fine particulate titanium oxide is often surface-treated by a fatty acid such as a stearic acid. A lot of fine particulate titanium oxides which are surface-treated by the aluminum hydroxide or the fatty acid and whose dispersibility into the oil agent is thereby enhanced are blended in W/O type emulsified cosmetics.

For example, it is described in Japanese Patent Application Laid-Open Publication No. 11-171541 (Patent Literature) is that first, coating treatment is conducted by using a water-soluble aluminum salt and a fine particulate titanium oxide obtained by conducting coating treatment using a fatty acid whose carbon number is 7 or more or its water-soluble salt is blended in a cosmetic.

On the other hand, in many O/W type emulsified cosmetics, for the purpose of a fresh use feeling, carbomer (carboxy vinyl polymer) is often blended. The carbomer imparts a thickening effect by mutual repulsion of negative electric charges in a carboxyl group in a structure of the carbomer. However, when the fine particulate titanium oxide surface-treated by the aluminum hydroxide is blended in the O/W type emulsified cosmetic, upon elution of aluminum ions, the eluted aluminum ions acts with the carbomer in terms of charge, thereby leading to a problem in that the thickening effect of the carbomer cannot be maintained.

Hence, it is preferable that in a case in which in the O/W type emulsified cosmetics, the fine particulate titanium oxide and the carbomer are used in combination, a fine particulate titanium oxide which is not treated by the aluminum hydroxide is used.

CITATION LIST

PATENT LITERATURE

Patent Literature 1: Japanese Patent Application Laid-Open Publication No. 11-171541

SUMMARY OF THE INVENTION

TECHNICAL PROBLEM

However, in order to suppress photoactivity of the fine particulate titanium oxide only by the treatment of the fatty acid, it is necessary to surface-treat the fine particulate titanium oxide by the fatty acid whose amount is excessively larger than an amount of the fatty acid which is needed to enhance dispersibility into the oil agent. In this case, since the excessive amount of the fatty acid is eluted into polar oil or the like in a pharmaceutical preparation, the eluted fatty acid reacts with other components and a by-product is thereby generated, thereby leading to a case in which the by-product exerts influence on viscosity and stability of the pharmaceutical preparation. On the other hand, if the amount of the fatty acid is kept to be an amount necessary to disperse the fine particulate titanium oxide to the oil agent, the photoactivity of the fine particulate titanium oxide cannot be sufficiently suppressed, a sufficient water-repellent property cannot be obtained, dispersibility upon blending into a pharmaceutical preparation is worsened, and stability of the pharmaceutical preparation is also worsened.

Therefore, an object of the present invention is to provide surface-treated powder of a fine particulate titanium oxide, which is capable of both suppressing photoactivity of the fine particulate titanium oxide and suppressing a treatment amount of a fatty acid in a compatible manner, enhancing effect to suppress photoactivity of the fine particulate titanium oxide, and enhancing stability of a pharmaceutical preparation.

SOLUTION TO PROBLEM

The present inventors have devoted themselves to earnest research. As a result, the present inventors have obtained the following findings. In the fine particulate titanium oxide, due to its small particle diameter, an amount of surface hydroxyl groups becomes large and in order to suppress photoactivity and to obtain dispersibility into an oil agent, a larger amount of a fatty acid is needed than in a case of a titanium oxide whose particle diameter is large. The present inventors have found that by setting a value of a product of a particle diameter of a fine particulate titanium oxide and a treatment amount of a fatty acid in a predetermined range, both of suppression of the photoactivity and suppression of a treatment amount of the fatty acid can be achieved in a compatible manner. By suppressing the treatment amount of the fatty acid, an elution amount of the fatty acid is suppressed and stability of a pharmaceutical preparation can be enhanced.

Based on the above-described findings, surface-treated powder of the present invention includes a fine particulate titanium oxide as powder to be surface-treated and a fatty acid, and a value of a product of an average primary particle diameter (nm) of the fine particulate titanium oxide and a ratio of a mass of the fatty acid to a mass of the fine particulate titanium oxide is 1.5 or more and 3.7 or less.

Thus, surface-treated powder of a fine particulate titanium oxide which is capable of suppressing the photoactivity of the fine particulate titanium oxide and enhancing stability of a pharmaceutical preparation can be provided.

DESCRIPTION OF EMBODIMENTS

Hereinafter, with reference to specific examples, surface-treated powder of the present invention and a cosmetic which includes the surface-treated powder will be described in detail. Note that the present invention is not limited to the below-described embodiments and a variety of modifications can be made without departing from the scope of the technical idea of the present invention.

1. Manufacturing of surface-treated powder

As one embodiment, surface-treated powder according to the present invention is manufactured by mixing a fine particulate titanium oxide and a fatty acid or a fatty acid salt and drying the resultant.

2. Powder to be surface-treated

The fine particulate titanium oxide as powder to be surface-treated is manufactured by the heretofore known method. A crystal form of the powder to be surface-treated is selected in accordance with an application. For example, in a case in which the fine particulate titanium oxide is blended in an emulsified cosmetic, it is preferable that the fine particulate titanium oxide includes a rutile type crystal. A particle diameter thereof is selected in accordance with an application and the like and for example, in a case in which the fine particulate titanium oxide is blended in an emulsified cosmetic, it is preferable that an average primary particle diameter is 5 nm or more and 50 nm or less, it is more preferable that the average primary particle diameter is 8 nm or more and 40 nm or less, and it is further preferable that the average primary particle diameter is 12 nm or more and 35 nm or less.

3. Fatty acid

The fatty acid is included in surface-treated powder as a hydrophobic treatment agent of the powder to be surface-treated. As the fatty acid, it is preferable that a stearic acid or its salt, a lauric acid or its salt, a myristic acid or its salt, a palmitic acid or its salt, or an isostearic acid or its salt is used and it is more preferable that a stearic acid or its salt or an isostearic acid or its salt is used. In addition, the fatty acid may be mixed and treated. Note that it is preferable that the salt of the fatty acid salt is alkali metal. It is more preferable that the salt thereof is sodium or potassium. It is more preferable that a value of a product between an average primary particle diameter (nm) of the fine particulate titanium oxide as the powder to be surface-treated in the surface-treated powder and a ratio of a mass of the fatty acid to a mass of the fine particulate titanium oxide is 1.5 or more and 3.7 or less or 2.0 or more and 3.0 or less. It is preferable that a content of the fatty acid in the surface-treated powder is 7% or more and 16% or less of a weight of the powder to be surface-treated in terms of the fatty acid and it is more preferable that the content thereof is 9% or more and 11% or less. A value of the content in terms of the fatty acid is a value calculated with a counter cation contained in the fatty acid or its salt excluded.

4. Other treatment

Although in the powder to be surface-treated, a metal hydrous oxide or a metal oxide can be treated, from a point of view of action of electrical charge with the carbomer, it is preferable that a treatment amount of the metal hydroxide or the metal oxide is small. In particular, it is preferable that alkaline-earth metal, aluminum, and zinc, which easily act with the carbomer in terms of electrical charge, are not treated practically. In a case in which the metal hydrous oxide or the metal oxide is used in the surface treatment, it is preferable that a treatment amount of the metal hydrous oxide or the metal oxide is 3% or less of a mass of the powder to be surface-treated (fine particulate titanium oxide), it is more preferable that the treatment amount thereof is 1% or less of the mass thereof, and it is further preferable that the metal hydrous oxide or the metal oxide is not treated practically. Furthermore, surface treatment may be conducted by using silane, a silane coupling agent, or silicone.

5. Cosmetic

The surface-treated powder of the present invention can be blended in cosmetics. The cosmetics in which the surface-treated powder thereof is blended are not limited and the surface-treated powder thereof may be blended in any of an O/W type emulsified cosmetic, a W/O emulsified cosmetic, and an oil type cosmetic.

In a case in which the surface-treated powder of the present invention is blended in the O/W type emulsified cosmetic, as a blending method, for example, the surface-treated powder of the present invention is added to a mixture of an oil phase raw material and while being agitated, a mixture of a water phase raw material can be added thereto.

[Examples]

With reference to specific manufacturing examples of cosmetics using the surface-treated powder according to the present invention and test results, further specific description will be given.

<Manufacturing of surface-treated powder>

Example 1

Step 1) Inputted into 35 kg of hydrous titanium dioxide cake obtained by filtrating and cleaning hydrolysate generated by heating an aqueous solution of titanyl sulfate crystal (manufactured by TAYCA CORPORATION, TM crystal) (a content of titanium dioxide: corresponding to 10 kg in terms of $TiO_2$) was 100 kg of a 48% sodium hydroxide aqueous solution while agitation is being conducted, the resultant was heated and was agitated at a range of 95°C to 105°C for 2 hours. Next, this suspension of the titanium dioxide hydrate was filtrated and the cake was sufficiently cleaned with water. Approximately 25 kg of water was added to the cleaned cake to be made into a slurry and the slurry was adjusted to be at 220 g/L of a concentration in terms of $TiO_2$. Furthermore, 1.7 kg of 35% hydrochloric acid was inputted thereto while being agitated and water was added thereto to be adjusted at 160 g/L in terms of $TiO_2$. Inputted thereto was 250 g of sodium sulfate anhydride and thereafter, 14.0 kg of 35% hydrochloric acid was inputted. Thermal aging was conducted at 95°C to 100°C for 2 hours. Solid particles in this slurry exhibited a crystal structure of a rutile-type titanium dioxide in X-ray diffraction.

Step 2)

A concentration of the obtained titanium dioxide aqueous suspension was adjusted to be 70 g/L. Ammonia water was added to 20 L of the aqueous suspension obtained as mentioned above (1.4 kg in terms of $TiO_2$), thereby adjusting a pH value to 6.0, the aqueous suspension was heated to 60°C and was aged, thereafter, the ammonia water was added again thereto, thereby adjusting the pH value to 6.0, and the slurry was filtrated and cleaned, thereby obtaining cleaned cake. The cake was dispersed in water again, was heated to 60°C, thereafter, the ammonia water was added thereto, thereby adjusting the pH value to 7.5, and aging therefor was conducted. After aging, the ammonia water was added thereto, thereby adjusting the pH value to 7.5 and the slurry was infiltrated and cleaned, thereby obtaining cleaned cake. The cleaned cake was put into a drier (150°C), thereby removing moisture, and titanium dioxide powder (dried product) was obtained.

Step 3)

The titanium dioxide powder (dried product) was baked at 400°C for 120 minutes and pulverizing was conducted, thereby obtaining fine particulate titanium oxide (burned product). An average primary particle diameter of the fine particulate titanium oxide (burned product) after pulverizing was 24.6 nm.

Step 4)

Water was added to the fine particulate titanium oxide (burned product), thereby adjusting a concentration to 100 g/L. Heated to 85°C was 10 L (1.0 kg in terms of $TiO_2$) of the obtained fine particulate titanium oxide (burned product) slurry.

Step 5)

After heating, 118 g of sodium stearate (11 weight% in terms of the fatty acid with respect to a mass of the fine particulate titanium oxide) was added to the slurry. At this time, the pH value gradually increased and 10 minutes later, became pH 7.0 and the slurry was aged for 1 hour. The pH value after the 1-hour aging was between 8.0 and 10.0.

Step 6)

Furthermore, after aging, the pH value of the slurry was adjusted to 6.7 by adding a sodium hydroxide aqueous solution or a sulfuric acid aqueous solution, thereafter, aging was conducted for 30 minutes, the slurry was filtrated and cleaned, drying at 105°C was conducted for 15 hours, the dried product was pulverized by an EC atomizer, thereby obtaining surface-treated powder in Example 1.

Example 2

Surface-treated powder was prepared in the same manner in which the surface-treated powder in Example 1 was prepared except that the sodium stearate in Step 5 was changed to sodium laurate.

Example 3

Surface-treated powder was prepared in the same manner in which the surface-treated powder in Example 1 was prepared except that the sodium stearate in Step 5 was changed to sodium myristate.

Example 4

Surface-treated powder was prepared in the same manner in which the surface-treated powder in Example 1 was prepared except that the sodium stearate in Step 5 was changed to sodium palmitate.

Example 5

Surface-treated powder was prepared in the same manner in which the surface-treated powder in Example 1 was prepared except that the sodium stearate in Step 5 was changed to isostearic acid.

Example 6

Surface-treated powder was prepared in the same manner in which the surface-treated powder in Example 1 was prepared except that the addition amount of the sodium stearate in Step 5 was changed to 75 g (7 weight% in terms of the fatty acid with respect to a mass of the fine particulate titanium oxide).

Example 7

Surface-treated powder was prepared in the same manner in which the surface-treated powder in Example 1 was prepared except that the addition amount of the sodium stearate in Step 5 was changed to 162 g (15 weight% in terms of the fatty acid with respect to a mass of the fine particulate titanium oxide).

Example 8

Surface-treated powder was prepared in the same manner in which the surface-treated powder in Example 1 was prepared except that in Step 3, roasting was conducted at a roasting temperature of 500°C, the average primary particle diameter of the obtained fine particulate titanium oxide was changed to 30.8 nm, and the addition amount of the sodium stearate was changed to 97 g (9 weight% in terms of the fatty acid with respect to a mass of the fine particulate titanium oxide).

Example 9

Surface-treated powder was prepared in the same manner in which the surface-treated powder in Example 1 was prepared except that in Step 3, roasting was conducted at a roasting temperature of 350°C for 120 minutes, the average primary particle diameter of the obtained fine particulate titanium oxide was changed to 23.6 nm, and the addition amount of the sodium stearate in Step 5 was changed to 153 g (14.2 weight% in terms of the fatty acid with respect to a mass of the fine particulate titanium oxide).

Comparative Example 1

Surface-treated powder was prepared in the same manner in which the surface-treated powder in Example 1 was prepared except that in Step 3, roasting was conducted at a roasting temperature of 350°C for 120 minutes, the average primary particle diameter of the obtained fine particulate titanium oxide was changed to 23.6 nm, and the addition amount of the sodium stearate in Step 5 was changed to 180 g (16.7 weight% in terms of the fatty acid with respect to a mass of the fine particulate titanium oxide).

Comparative Example 2

Surface-treated powder was prepared in the same manner in which the surface-treated powder in Example 1 was prepared except that the addition amount of the sodium stearate in Step 5 was changed to 32 g (3 weight% in terms of the fatty acid with respect to a mass of the fine particulate titanium oxide).

Comparative Example 3

Surface-treated powder was prepared in the same manner in which the surface-treated powder in Example 1 was prepared except that the addition amount of the sodium stearate in Step 5 was changed to 215 g (20 weight% in terms of the fatty acid with respect to a mass of the fine particulate titanium oxide).

<Calculation method of particle diameter>

As a particle diameter of the fine particulate titanium oxide, an average primary particle diameter of the fine particulate titanium oxide was measured after the pulverization in Step 3. The fine particulate titanium oxide was shot by a transmission electron microscope (TEM) and by using Particle size distribution analysis software (Mac-View: manufactured by Mountech Co., Ltd.), the particle diameter was calculated (a number of particles: 100). As a calculation method of the particle diameter, a Heywood diameter (projected area circle equivalent diameter) was adopted.

<Light resistance test>

Light resistance of the surface-treated powder in each of Examples and Comparative Examples was evaluated as follows. Measured into a 100 mL-polypropylene cup was 3.0 g of the fine particulate titanium oxide. Next, 4.0 g of 1,3-butylene glycol was measured. By using a Teflon rod, the fine particulate titanium oxide and the 1,3-butylene glycol were thoroughly mixed until enough blending was obtained, thereby preparing a paste. The paste was placed on film, a cover glass was put thereon so as to prevent oxygen from being supplied, and color tones $L_0$, $a_0$, and $b_0$ of each of the specimens were measured by a color and color difference meter (manufactured by Minolta Co., Ltd., CR-200). Each of these specimens was set on (Q-SLTN: Xe-1) and was irradiated with ultraviolet rays with an irradiance level of 60 W/m$^2$ and an integrated irradiance level of 100 kJ. After the irradiation of the defined level, as color tones $L_1$, $a_1$, and $b_1$ of each of the specimens, measurement in the above-described method was conducted again. A degree of discoloration ($\Delta E$) of each of the specimens was obtained by the following formula. The lower a value of $\Delta E$ is, the more excellent light resistance is. Results are shown in Table 1.

$$\Delta E=[(L_1-L_0)^2+(a_1-a_0)^2+(b_1-b_0)^2]^{1/2}$$

<Elution test>

An elution amount of the fatty acid in the surface-treated powder in each of Examples and Comparative Examples was evaluated as follows. Put into a 300-mL glass Erlenmeyer flask was 10 g of the surface-treated powder in each of Examples and Comparative Examples. Next, 50 g of acetone was measured, a cooling tube was connected thereto, and reflux was conducted in a hot water bath at 75°C for 10 minutes. After the reflux, by using a Kiriyama funnel and filter paper (5C), filtrating was repeated until filtrate became transparent. Inputted to the transparentized filtrate was 10 g of ion-exchanged water and 3 drops of a 1% phenolphthalein solution. Next, by using 0.1 M of a sodium hydroxide aqueous solution, titration was conducted. An eluted fatty acid amount (mmol) of each of the specimens was obtained by the following formula. Results are shown in Table 1.

Eluted fatty acid amount (mmol) = a 0.1 M sodium hydroxide aqueous solution amount (ml) used in the titration / 10

[Table 1]

| No. | Powder to be surface-treated | | Fatty acid | | Product of average primary particle diameter and ratio of mass of fine particulate titanium oxide and mass of fatty acid | Light resistance test | Elution test | Determination |
|---|---|---|---|---|---|---|---|---|
| | Powder | Average primary particle diameter (nm) | Kind | Content (%) | | $\Delta E$ | Eluted fatty acid amount (mmol) | |
| Example 1 | Titanium oxide | 24.6 | Stearic acid | 11 | 2.71 | 13.3 | 0.18 | Good |
| Example 2 | Titanium oxide | 24.6 | Lauric acid | 11 | 2.71 | 18.5 | 0.27 | Good |
| Example 3 | Titanium oxide | 24.6 | Myristic acid | 11 | 2.71 | 16.5 | 0.24 | Good |
| Example 4 | Titanium oxide | 24.6 | Palmitic acid | 11 | 2.71 | 14.2 | 0.28 | Good |
| Example 5 | Titanium oxide | 24.6 | Isostearic acid | 11 | 2.71 | 15.4 | 0.31 | Good |
| Example 6 | Titanium oxide | 24.6 | Stearic acid | 7 | 1.72 | 27.8 | 0.07 | Good |
| Example 7 | Titanium oxide | 24.6 | Stearic acid | 15 | 3.69 | 6.1 | 0.91 | Good |
| Example 8 | Titanium oxide | 30.8 | Stearic acid | 9 | 2.77 | 9.0 | 0.26 | Good |
| Example 9 | Titanium oxide | 23.6 | Stearic acid | 14.2 | 3.35 | 22.0 | 0.56 | Good |
| Comparative Example 1 | Titanium oxide | 23.6 | Stearic acid | 16.7 | 3.94 | 15.5 | 1.17 | Bad |
| Comparative Example 2 | Titanium oxide | 24.6 | Stearic acid | 3 | 0.74 | 39.5 | 0.06 | Bad |
| Comparative Example 3 | Titanium oxide | 24.6 | Stearic acid | 20 | 4.92 | 4.2 | 1.09 | Bad |

In Table 1, Examples and Comparative Examples whose $\Delta E$ was 30 or less and whose eluted fatty acid amount was 1 mmol or less were determined as "Good" and whose $\Delta E$ was above 30 or whose elution amount was larger than 1 mmol were determined as "Bad". As shown in Table 1, each of Examples 1 to 9 was determined as "Good" and both suppression of the photoactivity and suppression of elution of the fatty acid were achieved.

<Evaluation of emulsified cosmetic>

Example 10

By using the surface-treated powder in Example 1, an emulsified cosmetic whose surface-treated powder had 7 mass% of a concentration was prepared as follows and a viscosity, SPF, and UVAPF were evaluated. Inputted into a 100 mL polypropylene cup was 32.9 g of a mixture of the below-mentioned oil phase raw materials and by using a high-speed emulsifier and disperser "T.K. ROBOMICS" manufactured by PRIMIX Corporation, while agitation was being conducted at 1400 rpm, 4.9g of the surface-treated powder in each of Examples and Comparative Examples was added. Subsequently, the agitation speed was increased to 3000 rpm and agitation was conducted for 10 minutes. Thereafter, while the agitation was continued, 32.2 g of a mixture of the below-mentioned water phase raw materials was added and agitation was conducted at 3000 rpm for 5 minutes, thereby preparing an emulsified cosmetic in Example 10.

Comparative Example 4

An emulsified cosmetic in Comparative Example 4 was prepared by using the surface-treated powder in Comparative Example 1 and in the same method as in Example 10.

The oil phase raw materials of the emulsified cosmetic were as follows.

- 25.9 g of cyclopentasiloxane: "KF-995" manufactured by Shin-Etsu Chemical Co., Ltd.
- 3.5g of liquid paraffin: "MORESCO-WHITE P-70" manufactured by MORESCO Corporation
- 3.5g of PEG-9-dimeticone: "KF-6019" manufactured by Shin-Etsu Chemical Co., Ltd.

The water phase raw materials of the emulsified cosmetic were as follows.

- 22.4 g of ion-exchanged water
- 9.8 g of 1,3-butylene glycol

The viscosity of each of the emulsified cosmetics was measured by using DV-1+VISCOMETER manufactured by BROOKFIELD (rotor No. S63, a number of revolutions of 12 rpm). Results are shown in Table 2.

Measured were SPF and UVAPF of each of the emulsified cosmetics. A PMMA plate was used as a board and an amount of 1.3 mg/cm$^2$ of each of the emulsified cosmetics was uniformly applied onto the board and was naturally dried at room temperature for 30 minutes. By using an SPF analyzer (Optometrics Corporation, Labsphare UV-2000S), SPF and UVAPF were measured. Results are shown in Table 2.

[Table 2]

| No. | Surface-treated powder | Emulsified cosmetic | | |
|---|---|---|---|---|
| | | Viscosity (12 rpm) mPa•s | SPF | UVAPF |
| Example 10 | Example 1 | 260 | 17.5 | 6.2 |
| Comparative Example 4 | Comparative Example 1 | 710 | 11.1 | 4.9 |

Since the emulsified cosmetic in Example 10 includes the surface-treated powder in Example 1 in which the elution of the fatty acid are suppressed, as compared with the emulsified cosmetic in Comparative Example 4 which includes surface-treated powder in Comparative Example 1 in which the elution of the fatty acid are not suppressed, the viscosity of the emulsified cosmetic in Example 10 was suppressed to be a relatively low and UVA and UVB shielding ability was enhanced.

The embodiment disclosed as above and examples are to be considered in all respects only as illustrative and not restrictive. It is intended that the scope of the invention is, therefore, indicated by the appended claims rather than the foregoing description of the embodiments and examples and that all modifications and variations coming within the meaning and equivalency range of the appended claims are embraced within their scope.

**Claims**

1. Surface-treated powder including:

   a fine particulate titanium oxide as powder to be surface-treated; and
   a fatty acid, wherein
   a value of a product of an average primary particle diameter (nm) of the fine particulate titanium oxide and a ratio of a mass of the fatty acid (in terms of the fatty acid) to a mass of the fine particulate titanium oxide is 1.5 or more and 3.7 or less.

2. The surface-treated powder according to claims 1, wherein the mass of the fatty acid (in terms of the fatty acid) to the mass of the fine particulate titanium oxide is 7% or more and 16% or less.

3. A cosmetic including the surface-treated powder according to claim 1 or 2.

## INTERNATIONAL SEARCH REPORT

| International application No. |
| --- |
| **PCT/JP2024/018030** |

**A. CLASSIFICATION OF SUBJECT MATTER**

*A61K 8/29*(2006.01)i; *A61Q 17/04*(2006.01)i; *C01G 23/053*(2006.01)i
FI: A61K8/29; A61Q17/04; C01G23/053

According to International Patent Classification (IPC) or to both national classification and IPC

**B. FIELDS SEARCHED**

Minimum documentation searched (classification system followed by classification symbols)

A61K8/29; A61Q17/04; C01G23/053

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2011/007668 A1 (TAYCA CORPORATION) 20 January 2011 (2011-01-20) claims, paragraphs [0014], [0024]-[0032], [0040], examples 1-3, comparative examples 1-3 | 1-3 |
| X | JP 2022-176754 A (SAKAI CHEM. IND. CO., LTD.) 30 November 2022 (2022-11-30) claims, paragraphs [0015]-[0025], manufacturing examples 1, 7 | 1-3 |
| X | WO 2021/241444 A1 (DNP FINE CHEMICALS CO., LTD.) 02 December 2021 (2021-12-02) claims, paragraphs [0006], [0026]-[0033], [0037], particles 1, 2 | 1-3 |
| X | 微粒子酸化チタンMTシリーズ [online], テイカ株式会社, 26 May 2021, <URL: https://www.tayca.co.jp/lp/assets/pdf/pdf_no9.pdf>, [retrieved on 04 July 2024], (MT series grade map of micro titanium dioxide, TAYCA CORPORATION) particularly, columns of MT-200ST, MT-200B | 1-3 |
| P, X | WO 2024/004579 A1 (SHIN-ETSU CHEMICAL CO., LTD.) 04 January 2024 (2024-01-04) claims, paragraphs [0013]-[0016], examples 1-3, 5-12, 14-20, comparative examples 1-14, 24-26 | 1-3 |

☑ Further documents are listed in the continuation of Box C.     ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **04 July 2024** | **16 July 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2024/018030**

**C.    DOCUMENTS CONSIDERED TO BE RELEVANT**

| Category* | Citation of document, with indication, where appropriate, of the relevant passages | Relevant to claim No. |
|---|---|---|
| A | MICRO TITANIUM DIOXIDE MT-200ST, テイカ株式会社, 17 April 2012, <URL: https://www.tayca.co.jp/products/pdf/20120417.pdf> [online], [retrieved on 04 July 2024], (TAYCA CORPORATION)<br>particularly, table of composition | 1-3 |
| A | JP 2021-521077 A (CRODA INTERNATIONAL PLC) 26 August 2021 (2021-08-26)<br>whole document | 1-3 |

Form PCT/ISA/210 (second sheet) (July 2022)

## INTERNATIONAL SEARCH REPORT
### Information on patent family members

International application No.

**PCT/JP2024/018030**

| Patent document cited in search report | | | Publication date (day/month/year) | Patent family member(s) | | | Publication date (day/month/year) |
|---|---|---|---|---|---|---|---|
| WO | 2011/007668 | A1 | 20 January 2011 | US | 2012/0100196 | A1 | |
| | | | | claims, paragraphs [0032]-[0033], [0043]-[0055], examples 1-3, comparative examples 1-3 | | | |
| | | | | EP | 2455061 | A1 | |
| | | | | AU | 2010271992 | A | |
| | | | | CN | 102470090 | A | |
| | | | | KR | 10-2012-0046150 | A | |
| JP | 2022-176754 | A | 30 November 2022 | (Family: none) | | | |
| WO | 2021/241444 | A1 | 02 December 2021 | (Family: none) | | | |
| WO | 2024/004579 | A1 | 04 January 2024 | (Family: none) | | | |
| JP | 2021-521077 | A | 26 August 2021 | US | 2021/0039957 | A1 | |
| | | | | whole document | | | |
| | | | | WO | 2019/197575 | A1 | |
| | | | | EP | 3774658 | A1 | |
| | | | | CN | 111918836 | A | |
| | | | | KR | 10-2020-0141456 | A | |
| | | | | BR | 112020020750 | A2 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11171541 A